Europäisches Patentamt

European Patent Office

Office européen des brevets

(19)

(11) Numéro de publication : **0 363 253 B1**

(12)

# FASCICULE DE BREVET EUROPEEN

(45) Date de publication du fascicule du brevet :
**22.04.92 Bulletin 92/17**

(51) Int. Cl.⁵ : **B01J 29/20,** C10G 45/64, C07C 5/27

(21) Numéro de dépôt : **89402635.0**

(22) Date de dépôt : **26.09.89**

(54) **Catalyseur à base de mordénite renfermant au moins un métal du groupe VIII et son utilisation en isomérisation d'une coupe C8 aromatique.**

(30) Priorité : **05.10.88 FR 8813145**

(43) Date de publication de la demande :
**11.04.90 Bulletin 90/15**

(45) Mention de la délivrance du brevet :
**22.04.92 Bulletin 92/17**

(84) Etats contractants désignés :
**BE DE ES GB IT NL**

(56) Documents cités :
**EP-A- 0 196 965
FR-A- 2 364 879
US-A- 232 181
US-A- 3 673 267**

(73) Titulaire : **INSTITUT FRANCAIS DU PETROLE
4, Avenue de Bois Préau
F-92502 Rueil-Malmaison (FR)**

(72) Inventeur : **Travers, Christine
12, Bd du Général De Gaulle
F-92500 Rueil Malmaison (FR)**
Inventeur : **Raatz, Francis
10, Allée Jacques Prévert
F-78260 Acheres (FR)**
Inventeur : **Marcilly, Christian
91ter rue Condorcet
F-78800 Houilles (FR)**
Inventeur : **Ribeiro Ramoa, Fernando
Rua Manuel Ferreira de Andrade 13-7-Esq.
Lisboa (PT)**
Inventeur : **Gomes Ribeiro, Maria Filipa
Expanseao Poente.Lote 29-1.DTO
2 700 Amadora (PT)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un catalyseur de type alumino-silicate comprenant une mordénite, dont on a modifié de manière contrôlée, par des traitements thermique(s) et/ou chimique(s) la taille des canaux et par conséquent la sélectivité géométrique, au moins un métal du groupe VIII de la classification périodique des éléments (Handbook of Chemistry and Physics, 6ème édition, 1980-81), éventuellement une matrice et son utilisation dans les réactions d'isomérisation des hydrocarbures $C_8$ aromatiques.

Actuellement les catalyseurs utilisés industriellement dans ces réactions sont essentiellement la zéolithe ZSM5, seule ou éventuellement en mélange avec d'autres zéolithes, telles que la mordénite par exemple. Ces catalyseurs sont notamment décrits dans les brevets US-A-4 467 129, 4 482 773 et EP-B-0138617.

L'intérêt de la ZSM5 réside dans son excellente sélectivité de forme, qui conduit à une grande sélectivité en paraxylène, due à la taille de ses canaux qui ne laissent passer que les xylènes, empêchant notamment la formation des triméthylbenzènes ; néanmoins, elle possède une activité relativement faible.

La mordénite est une zéolithe très active, notamment dans les réactions d'isomérisation des coupes $C_8$ aromatiques, en particulier plus active que les zéolithes de structure MFI ; mais elle est peu sélective, la réaction de dismutation des xylènes en triméthylbenzènes et en toluène étant très importante. Grâce à la présente invention, il est notamment possible d'obtenir par des traitements thermique(s) et/ou chimique(s) (acide(s)) appropriés une mordénite qui présente des performances en isomérisation (en particulier un rendement en isomérisation) au moins équivalentes à celles des catalyseurs de structure MFI, et avec laquelle, par conséquent, les réactions secondaires, telles que la dismutation en particulier, sont considérablement réduites.

La zéolithe utilisée dans le catalyseur de la présente invention est fabriquée à partir d'une mordénite dite "petits pores" dont la teneur pondérale en sodium est comprise généralement entre 4 et 6,5 %, dont le rapport atomique Si/Al de charpente est compris généralement entre 4,5 et 6,5 et le volume de maille élémentaire généralement compris entre 2,77 et 2,80 nm³ (avec 1 nm = $10^{-9}$ m). Cette mordénite n'adsorbe habituellement que des molécules de diamètre cinétique inférieur à environ 4,4 x $10^{-10}$ m.

Cette mordénite petits pores est tout d'abord soumise à une réaction d'échange permettant de remplacer les cations sodium par des cations ammonium. Cet échange est réalisé en plongeant la mordénite dans une solution d'un sel d'ammonium ionisable, par exemple le nitrate d'ammonium, de molarité généralement supérieure à 0,5, à une température comprise habituellement entre 20 et 150°C. Cette étape d'échange peut être répétée plusieurs fois, et peut éventuellement être suivie d'une ou plusieurs étapes de lavage. La teneur pondérale en sodium de la mordénite ainsi obtenue est en général inférieure à 0,2 % et, de préférence, inférieure à 0,12 % ; son volume de maille élémentaire et son rapport atomique Si/Al restent sensiblement inchangés ; sa capacité d'adsorption de benzène est de l'ordre de 1 % poids par rapport au poids de mordénite sèche, et elle n'adsorbe que très peu les triméthylbenzènes. Sa teneur pondérale en eau est comprise entre 5 et 40 % et, de préférence, entre 10 et 30 %.

Selon l'invention, la mordénite obtenue, ayant la teneur pondérale en eau indiquée ci-dessus, doit ensuite subir divers traitements et en particulier :
– soit au moins une calcination sous courant d'air sec, suivie ou non d'au moins une attaque acide,
– soit au moins une attaque acide directe pour notamment déboucher au moins partiellement sa structure.
. On entend par calcination sous courant d'air sec, un traitement thermique, réalisé dans des conditions bien précises de température, de teneur en eau de l'air et de débit d'air, qui permet de laisser la vapeur d'eau dégagée par le solide suffisamment en contact avec ledit solide pour qu'elle puisse agir sur la structure et désaluminer plus ou moins la charpente selon la température de calcination utilisée. Cette désalumination de la charpente par élimination continue des atomes d'aluminium en position tétraédrique provoque, dans le cas de la mordénite à petits pores utilisée dans l'invention, un débouchage de la porosité que l'on peut maîtriser en contrôlant la température de calcination.

Les conditions de calcination préférentiellement utilisées dans la présente invention sont les suivantes :
– une teneur pondérale en eau de l'air (avant son contact avec le solide) inférieure à 1 % et, de préférence, inférieure à 300 ppm,
– un débit d'air compris entre 0,5 et 10 litres par heure et par gramme de solide et, de préférence, entre 1 et 5 litres par heure et par gramme de solide (1/h/g),
– une température de calcination comprise entre environ 450 et environ 650°C et, de préférence, entre environ 450 et environ 580°C,
– une vitesse de montée en température comprise entre 2 et 8°C par minute et, de préférence, entre 3 et 6°C par minute.

La température de calcination finale sera généralement maintenue pendant environ 0,5 à environ 5 heures et, de préférence, pendant environ 1 à environ 4 heures.

Le traitement thermique de calcination sous courant d'air sec peut éventuellement être suivi d'au moins une attaque acide (douce) permettant d'éliminer les ions aluminium en position octaédrique sans toucher aux ions aluminium de charpente pour cela, le solide, après calcination, est chauffé dans une solution d'un acide minéral ou organique, tel que par exemple l'acide chlorhydrique, l'acide nitrique, de normalité habituellement inférieure à 2N et, de préférence, inférieure à 0,5 N, à une température inférieure à environ 100°C et, de préférence, inférieure à environ 60°C, en général pendant 4 heures environ, avec un rapport $H_s^+/Al_z$ compris entre 8,5 et 11,5 et, de préférence, égal à environ 10, où $H_s^+$ est le nombre de moles de protons en solution et $Al_z$ le nombre de moles de cations aluminium dans la mordénite.

. La mordénite selon l'invention peut également être obtenue par au moins une attaque acide directe, qui consiste à chauffer à une température supérieure à environ 70°C et, de préférence, supérieure à environ 85°C, la zéolithe après l'étape d'échange cationique (et éventuellement de lavage) dans une solution d'un acide minéral ou organique, tel que par exemple l'acide chlorhydrique, l'acide nitrique, de normalité habituellement comprise entre 0,1 et 5N et, de préférence, entre 0,2 et 3N, pendant en général 2 heures environ, avec un rapport $H_s^+/Al_z$ compris entre 5 et 20 et, de préférence, entre 8 et 15, où $H_s^+$ est le nombre de moles de protons dans la solution et $Al_z$ le nombre de moles de cations aluminium dans la mordénite. Il se produit alors une extraction des cations aluminium tant en position tétraédrique qu'octaédrique et donc un déblocage de la porosité d'autant plus importante que l'attaque acide est forte.

La mordénite obtenue par l'un des traitements précédents a un volume de maille élémentaire compris entre 2,73 et 2,78 nm³. Son rapport atomique Si/Al de charpente est compris entre 6 et 10,5 et, de préférence, entre 6 et 9,5. La teneur pondérale en sodium de ladite mordénite est en général inférieure à 2000 ppm et, de préférence, inférieure à 1000 ppm. Sa capacité d'adsorption de benzène varie de 4 à 10 %, de préférence de 5 à 9 %, poids par rapport au poids de mordénite sèche et sa capacité d'adsorption de 1,3,5 triméthylbenzène de 0,5 à 2,5 %, de préférence de 0,7 à 2 %, poids par rapport au poids de mordénite sèche.

Les mordénites contenues dans les catalyseurs de l'invention ont donc la particularité de pouvoir adsorber des quantités relativement importantes de benzène, mais de ne très peu adsorber le 1,3,5 triméthylbenzène, qui est un produit formé lors de la réaction indésirable de dismutation des xylènes. Leurs capacités d'adsorption sont en général déterminées par gravimétrie, par exemple selon la méthode ci-après : un échantillon de la mordénite est préalablement désorbé à 300°C sous $10^{-4}$ Torr (133,32x$10^{-4}$ Pa), puis l'adsorption est menée pendant au moins 4 heures dans les conditions suivantes :

– pour l'adsorption de benzène :

T = 30°C

P = 28 Torr (3733 Pa)

P/Ps = 0,25

– pour l'adsorption de 1,3,5 triméthylbenzène :

T = 50°C

P = 3 Torr (400 Pa)

P/Ps = 0,26

où Ps représente la pression de vapeur saturante à la température de l'expérience. Les volumes adsorbés sont calculés à partir de la densité de l'adsorbat sous forme liquide à la température de l'adsorption : $d_o$ = 0,868 pour le benzène et $d_o$ = 0,839 pour le 1,3,5 triméthylbenzène.

Les autres caractéristiques peuvent être mesurées par les méthodes suivantes :

– les rapports atomiques Si/Al de charpente sont déterminés par spectrométrie infra-rouge, les teneurs en sodium par adsorption atomique,

– le volume de maille élémentaire est déterminé par diffraction X, l'échantillon de mordénite étant préparé de manière analogue au mode opératoire de la norme ASTM D 3942 80 établie pour la faujasite.

La mordénite ainsi modifiée peut être soumise telle quelle au dépôt d'au moins un métal du groupe VIII, de préférence choisi dans le groupe formé par le platine et le palladium, et mise en forme par toutes les techniques connues de l'homme du métier. Elle peut en particulier être mélangée à une matrice, généralement amorphe, par exemple à une poudre humide de gel d'alumine. Le mélange est ensuite mis en forme, par exemple par extrusion au travers d'une filière. La teneur en mordénite du support (mordénite + matrice) ainsi obtenu est généralement comprise entre environ 0,5 et 99,99 % et avantageusement comprise entre environ 40 et 90 % en poids par rapport au support. Elle est plus particulièrement comprise entre environ 60 et 85 % en poids par rapport au support. La teneur en matrice du catalyseur est avantageusement comprise entre environ 10 et 60 % et de préférence entre environ 15 et 40 % en poids par rapport au support (mordénite + matrice).

La mise en forme peut être réalisée avec d'autres matrices que l'alumine, telles que, par exemple, la magnésie, la silice-alumine, les argiles naturelles (kaolin, bentonite), et par d'autres techniques que l'extrusion, telles que le pastillage ou la dragéification.

Le métal hydrogénant du groupe VIII, de préférence Pt et/ou Pd, est ensuite déposé sur le support par tout procédé connu de l'homme du métier et permettant le dépôt du métal sur la mordénite. On peut utiliser la technique d'échange cationique avec compétition, où l'agent compétiteur est de préférence le nitrate d'ammonium, le rapport de compétition étant au moins égal à environ 50, et avantageusement d'environ 50 à 200. Dans le cas du platine ou du palladium, on utilise habituellement un complexe tétrammine du platine ou un complexe tétrammine du palladium ; ces derniers se déposeront alors pratiquement en totalité sur la mordénite. Cette technique d'échange cationique peut également être utilisée pour déposer directement le métal sur la poudre de mordénite, avant son mélange éventuel avec une matrice.

Le dépôt du métal (ou des métaux) est suivi en général d'une calcination sous air ou oxygène, usuellement entre 300 et 600°C durant 0,5 à 10 heures, de préférence entre 350 et 550°C durant 1 à 4 heures. On peut procéder ensuite à une réduction sous hydrogène, généralement à une température comprise entre 300 et 600°C pendant 1 à 10 heures. De préférence, on opèrera entre 350 et 550°C pendant 2 à 5 heures. La teneur en métal du groupe VIII (Pt et/ou Pd) déposé sur le catalyseur et obtenue à l'issue de l'échange se situe entre 0,05 et 1,5 %, de préférence entre 0,1 et 1 %, en poids par rapport à l'ensemble du catalyseur.

On peut également déposer le platine ou le palladium non plus directement sur la mordénite, mais sur le liant aluminique, avant ou après l'étape de mise en forme, en mettant en oeuvre un échange anionique avec de l'acide hexachloroplatinique, de l'acide hexachloropalladique et/ou du chlorure de palladium en présence d'un agent compétiteur, par exemple l'acide chlorhydrique. Après dépôt de platine et/ou palladium, le catalyseur est comme précédemment soumis à une calcination, en général entre 300 et 600°C, puis réduit sous hydrogène comme indiqué précédemment.

Le catalyseur bifonctionnel obtenu par les procédures précédentes peut être mis en oeuvre notamment dans les réactions d'isomérisation d'une coupe $C_8$ aromatique, comprenant par exemple soit uniquement un mélange de xylènes, soit un mélange de xylènes et d'éthylbenzène. L'isomérisation des alkyl-aromatiques, et en particulier des xylènes, revêt une importance commerciale considérable. C'est en général surtout le paraxylène qui est le produit le plus recherché, car il est notamment utilisé comme intermédiaire dans la fabrication des fibres polyester. On préfère fabriquer le paraxylène en isomérisant le métaxylène, qui lui peut être obtenu par isomérisation de l'orthoxylène. L'éthylbenzène, qui est difficilement séparable par distillation (les points d'ébullition des différents composés sont très proches) du mélange des xylènes, se trouve très souvent dans la charge d'isomérisation des $C_8$ aromatiques.

Les conditions opératoires du procédé d'isomérisation sont généralement les suivantes :
– température comprise entre 240 et 600°C, de préférence entre 350 et 510°C,
– pression comprise entre 0,5 et 100 bars, de préférence entre 2 et 30 bars,
– vitesse spatiale (pph), en masse de charge par unité de charge de catalyseur et par heure, comprise entre 0,5 et 200, de préférence entre 5 et 100,
– rapport molaire hydrogène sur hydrocarbures de la charge ($H_2$/HC) compris entre 0,5 et 12, de préférence entre 2 et 6.

Les exemples qui suivent illustrent l'invention sans toutefois en limiter la portée ; ils sont donnés pour une charge formée de 75 % d'orthoxylène et de 25 % d'éthylbenzène (% en poids).

EXEMPLE 1 : Catalyseur A conforme à l'invention.

La matière première est une mordénite "petits pores", référence Alite 150 de la Société Chimique de la Grande Paroisse. Sa formule chimique à l'état anhydre est : Na $AlO_2(SiO_2)_{5,5}$, et sa capacité d'adsorption de benzène est de 1 % en poids par rapport au poids de solide sec (volume de maille : 2,79 nm³ ; teneur en sodium 5,3 % (poids) ; diamètre cinétique des molécules adsorbées : $3,8 \times 10^{-10}$ m) ; 50 grammes de cette poudre sont plongés dans une solution 2M de nitrate d'ammonium et la suspension est portée à 95°C pendant deux heures.

Le volume de la solution de nitrate d'ammonium engagée est égal à 4 fois le poids de zéolithe sec (V/P = 4 cm³/g). Cette opération d'échange cationique est recommencée 3 fois. Après le 3ème échange, le produit est lavé à l'eau, à 20°C pendant 20 minutes, avec un rapport V/P égal à 4. La teneur en sodium, exprimée en pourcentage en poids par rapport au poids de solide sec, passe de 5,5 à 0,1 %. Le produit est ensuite filtré et soumis à une calcination sous courant d'air sec dont la teneur pondérale en eau est inférieure à 300 ppm, à environ 550°C pendant 2 heures.

Pour cela, 2 grammes de poudre (mordénite dont la teneur pondérale en eau est d'environ 25 %)) sous forme ammonium sont chargés dans un réacteur en quartz de 30 mm de diamètre. Le lit de poudre formé est traversé par un débit d'air sec égal à 2 litres par heure. La vitesse de montée en température est de 4,2°C par minute. Une fois la température de 550°C atteinte, on maintient cette température en palier pendant 2 heures.

La mordénite ainsi obtenue possède un rapport atomique Si/Al de charpente de 7,5. Sa teneur pondérale en sodium est de 1000 ppm. Son volume de maille élémentaire est égal à 2,76 nm³. Sa capacité d'adsorption de benzène est de 8 % poids par rapport au poids de mordénite sèche et sa capacité d'adsorption de 1,3,5 triméthylbenzène de 1,8 % poids par rapport au poids de mordénite sèche.

On mélange ensuite intimement cette mordénite avec de l'alumine sur laquelle on a dispersé 0,3 % en poids de platine, le support constitué par le mélange mordénite-alumine contenant 40 % en poids d'alumine. La teneur pondérale en platine du catalyseur final A est donc de 0,12 %.

Le catalyseur ainsi fabriqué est ensuite mis en forme par pastillage, calciné sous air jusqu'à 500°C durant 2 heures et réduit sous hydrogène à 500°C pendant 3 heures.

Ce catalyseur A est alors testé en isomérisation du mélange orthoxylène (75 % poids) et éthylbenzène (25 % poids), à une température de 420°C, sous une pression 15 bars, avec une vitesse spatiale (pph) de 50 (heure)$^{-1}$ et un rapport molaire hydrogène sur hydrocarbures (H$_2$/HC) de 4 environ.

Les performances du catalyseur A (et des catalyseurs préparés dans les exemples suivants), reportées dans le tableau I, sont définies par :

$$\text{Conversion de l'o-xylène(\%)} = \frac{(\text{Masse d'o-xylène dans la charge})-(\text{masse d'o-xylène dans la recette})}{\text{Masse d'o-xylène dans la charge}} \times 100$$

$$\text{Sélectivité en isomération(\%)} = \frac{(\text{Masse de m-xylène}) + (\text{masse p-xylène})}{\text{Masse des produits}} \times 100$$

$$\text{Rendement en isomération \%} = \frac{\text{Conversion} \times \text{sélectivité}}{100}$$

$$\text{Sélectivité en dismutation (\%)} =$$
$$\frac{(\text{Masse de triméthylbenzène}) + (\text{masse de toluène}) + (\text{masse de benzène})}{\text{Masse des produits}} \times 100$$

$$\text{Sélectivité en craquage (\%)} = \frac{\text{Masse des gaz de C1 à C4}}{\text{Masse des produits}} \times 100$$

EXEMPLE 2 : Catalyseur B conforme à l'invention.

Le catalyseur B diffère du catalyseur A préparé dans l'exemple 1 en ce qu'après l'étape de calcination sous courant d'air sec, la mordénite est soumise à une attaque acide douce consistant à chauffer ladite mordénite dans une solution d'acide chlorhydrique 0,2 N à 50°C pendant 4 heures, avec un rapport H$_s^+$/Al$_z$ égal à 10 environ.

La mordénite ainsi obtenue possède un rapport atomique Si/Al de charpente de 7,5. Sa teneur pondérale en sodium est inférieure à 300 ppm. Son volume de maille élémentaire est égal à 2,76 nm$^3$. Sa capacité d'adsorption de benzène est de 8 % poids par rapport au poids de mordénite sèche et sa capacité d'adsorption de 1,3,5 triméthylbenzène de 1,8 % poids par rapport au poids de mordénite sèche.

Les étapes de mélange mordénite-alumine, de dispersion du platine, de mise en forme, de calcination, de réduction du catalyseur et les conditions de test en isomérisation sont identiques à celles décrites dans l'exemple 1.

Les performances du catalyseur B sont reportées dans le tableau I.

EXEMPLE 3 : Catalyseur C conforme à l'invention.

Le catalyseur C diffère du catalyseur A préparé dans l'exemple 1 en ce qu'après le 3ème échange cationique (et le lavage à l'eau) le solide est directement soumis à une attaque acide : il est porté à reflux dans une solution d'acide chlorhydrique 0,3 N à 90°C pendant 2 heures, avec un rapport H$_s^+$/Al$_z$ égal à 10 environ.

La mordénite ainsi obtenue possède un rapport atomique Si/Al de charpente de 7,6. Sa teneur pondérale en sodium est inférieure à 300 ppm. Son volume de maille élémentaire est égal à 2,76 nm$^3$. Sa capacité d'adsorption de benzène est de 8,1 % poids par rapport au poids de mordénite sèche et sa capacité d'adsorption de 1,3,5 triméthylbenzène de 1,75 % poids par rapport au poids de mordénite sèche.

Les étapes de mélange mordénite-alumine, de dispersion du platine, de mise en forme, de calcination, de réduction du catalyseur et les conditions de test en isomérisation sont identiques à celles décrites dans l'exemple 1.

Les performances du catalyseur C sont reportées dans le tableau I.

EXEMPLE 4 : Catalyseur D non conforme à l'invention.

Le catalyseur D renferme une zéolithe de structure MFI, synthétisée en milieu fluorure ; cette zéolithe possède un rapport atomique Si/Al de charpente de 250. Sa teneur pondérale en sodium est de 50 ppm. Son volume de maille élémentaire est égal à 5,36 nm$^3$. Sa capacité d'adsorption de benzène est de 9,5 % poids par rapport au poids de zéolithe sèche et sa capacité d'adsorption de 1,3,5 triméthylbenzène de 1,5 % poids par rapport au poids de zéolithe sèche.

Les étapes de mélange zéolithe-alumine, de dispersion du platine, de mise en forme, de calcination, de réduction du catalyseur et les conditions de test en isomérisation sont identiques à celles décrites dans l'exemple 1, mais avec une pph de 30 (heures)$^{-1}$.

Les performances du catalyseur D sont reportées dans le tableau I.

EXEMPLE 5 : Catalyseur E non conforme à l'invention.

Le catalyseur E (décrit dans la demande de brevet européen EP-A-196965) diffère du catalyseur A préparé dans l'exemple 1 en ce qu'après le 3ème échange cationique (et le lavage à l'eau), le solide est filtré, puis soumis à une calcination en atmosphère confinée ("self-steaming") à 600°C pendant 2 heures (l'atmosphère de calcination contenant au moins 5 % de vapeur d'eau). On procède ensuite à une attaque acide : le solide est porté à reflux dans une solution d'acide chlorhydrique 0,6N à 90°C pendant 2 heures, avec un rapport V/P égal à 8, où V est le volume de la solution d'acide chlorhydrique et P le poids de mordénite sèche. Le produit est ensuite filtré, lavé à l'acide chlorhydrique 0,1N puis à l'eau.

La mordénite ainsi obtenue possède un rapport atomique Si/Al de charpente de 12. Sa teneur pondérale en sodium est d'environ 300 ppm. Son volume de maille élémentaire est égal à 2,75 nm$^3$. Sa capacité d'adsorption de benzène est de 9,6 % poids par rapport au poids de mordénite sèche et sa capacité d'adsorption de 1,3,5 triméthylbenzène de 8 % poids par rapport au poids de mordénite sèche, ce qui signifie que la mordénite "petits pores" est totalement débouchée et équivalente à une mordénite "larges pores" du point de vue capacités d'adsorption.

Les étapes de mélange mordénite-alumine, de dispersion du platine, de mise en forme, de calcination, de réduction du catalyseur et les conditions de test en isomérisation sont identiques à celles décrites dans l'exemple 1.

Les performances du catalyseur E sont reportées dans le tableau I.

Les catalyseurs A, B, C conformes à l'invention sont plus performants que les catalyseurs D et E de l'art antérieur : le rendement en isomérisation des catalyseurs A, B, C est supérieur à celui des catalyseurs D et E.

En effet, le catalyseur D est beaucoup moins actif que les catalyseurs A, B, C, et le catalyseur E est très peu sélectif.

TABLEAU I

| CATALYSEUR | A | B | C | D | E |
|---|---|---|---|---|---|
| Exemple | 1 | 2 | 3 | 4 | 5 |
| pph ($h^{-1}$) | 50 | 50 | 50 | 30 | 50 |
| Conversion (%) de l'o-xylène | 58,0 | 57,5 | 58,0 | 50,0 | 79,5 |
| Sélectivité en isomérisation (%) | 80,0 | 81,2 | 79,5 | 91,5 | 27,0 |
| Rendement en isomérisation (%) | 46,4 | 46,7 | 46,1 | 45,7 | 21,5 |
| Sélectivité en dismutation (%) | 6,5 | 6,6 | 6,4 | 5,5 | 55,0 |
| Sélectivité en craquage (%) | 1,5 | 1,5 | 1,6 | 0,8 | 1,7 |

**Revendications**

1. Catalyseur contenant une mordénite et au moins un métal du groupe VIII de la classification périodique des éléments caractérisé en ce que ladite mordénite est telle que :
   – son rapport atomique Si/Al de charpente est compris entre 6 et 10,5,
   – sa teneur pondérale en sodium est inférieure à 2000 ppm,
   – son volume de maille élémentaire est compris entre 2,73 et 2,78 nm³,
   – sa capacité d'absorption de benzène est comprise entre 4 et 10% poids par rapport au poids de mordénite sèche,
   – sa capacité d'absorption de 1,3,5 triméthylbenzène est comprise entre 0,5 et 2,5 % poids par rapport au poids de mordénite sèche.

2. Catalyseur selon la revendication 1 caractérisé en ce que ladite mordénite est telle que :
   – sa capacité d'absorption de benzène est comprise entre 5 et 9 % poids par rapport au poids de mordénite sèche,
   – sa capacité d'absorption de 1,3,5 triméthylbenzène est comprise entre 0,7 et 2 % poids par rapport au poids de mordénite sèche.

3. Catalyseur selon l'une des revendications 1 et 2 caractérisé en ce que ladite mordénite est telle que :
– son rapport atomique Si/Al de charpente est compris entre 6 et 9,5,
– sa teneur pondérale en sodium est inférieure à 1000 ppm.

4. Catalyseur selon l'une des revendications 1 à 3 dans lequel ledit métal est choisi dans le groupe formé par le platine et le palladium.

5. Catalyseur selon l'une des revendications 1 à 4 caractérisé en ce qu'il renferme en outre une matrice.

6. Procédé de préparation d'une mordénite contenue dans un catalyseur selon l'une des revendications 1 à 5 à partir d'une mordénite dite "petits pores" dont le rapport atomique Si/Al de charpente est compris entre 4,5 et 6,5, dont la teneur pondérale en sodium est comprise entre 4 et 6,5 %, dont le volume de maille élémentaire est compris entre 2,77 et 2,80 nm³, ladite mordénite "petits pores" n'absorbant que des molécules de diamètre cinétique inférieur à environ $4,4 \times 10^{-10}$ m, le procédé étant caractérisé en ce que :
a) on procède au moins une fois à un échange des cations sodium de la mordénite dite "petits pores" par des cations ammonium,
b) on fait subir au solide obtenu à l'étape a) une calcination sous courant d'air sec dont la teneur pondérale en eau est inférieure à 1 %, sous un débit d'air compris entre 0,5 et 10 litres par heure et par gramme de solide, à une température comprise entre environ 450 et 650°C, avec une vitesse de montée en température comprise entre 2 et 8°C par minute, la température de calcination finale étant maintenue pendant environ 0,5 à environ 5 heures.

7. Procédé selon la revendication 6 dans lequel le solide obtenu à l'étape b) est chauffé dans une solution d'un acide minéral ou organique de normalité inférieure à 2N, à une température inférieure à environ 100°C, avec un rapport $H_s^+/Al_z$ compris entre 8,5 et 11,5, où $H_s^+$ est le nombre de moles de protons en solution et $Al_z$ le nombre de moles de cations aluminium dans la mordénite.

8. Procédé de préparation d'une mordénite contenue dans un catalyseur selon l'une des revendications 1 à 5 à partir d'une mordénite dite "petits pores" dont le rapport atomique Si/Al de charpente est compris entre 4,5 et 6,5, dont la teneur pondérale en sodium est comprise entre 4 et 6,5 %, dont le volume de maille élémentaire est compris entre 2,77 et 2,80 nm³, ladite mordénite "petits pores" n'absorbant que des molécules de diamètre cinétique inférieur à environ $4,4 \times 10^{-10}$ m, le procédé étant caractérisé en ce que :
a) on procède au moins une fois à un échange des cations sodium de la mordénite dite "petits pores" par des cations ammonium,
b) on chauffe le solide obtenu à l'étape a) à une température supérieure à environ 70°C dans une solution d'un acide minéral ou organique de normalité comprise entre 0,1 et 5N, avec un rapport $H_s^+/Al_z$ compris entre 5 et 20, où $H_s^+$ est le nombre de moles de protons dans la solution et $Al_z$ le nombre de moles de cations aluminium dans la mordénite.

9. Utilisation d'un catalyseur selon l'une des revendications 1 à 5 dans les réactions d'isomérisation d'une coupe $C_8$ aromatique.

## Claims

1. Catalyst containing a mordenite and at least one metal from group VIII of the periodic classification of elements, characterized in that
– the said mordenite is such that its skeleton Si/Al atomic ratio is between 6 and 10.5,
– its sodium weight content is below 2000 ppm,
– its unit mesh volume is between 2.73 and 2.78 nm³,
– its benzene adsorption capacity is between 4 and 10% by weight,
– based on the dry mordenite weight and its 1,3,5-trimethylbenzene adsorption capacity is between 0.5 and 2.5% by weight, based on the dry mordenite weight.

2. Catalyst according to claim 1, characterized in that
– said mordenite is such that its benzene absorption capacity is between 5 and 9% by weight,
– based on the dry mordenite weight and its 1,3,5-trimethylbenzene adsorption capacity is between 0.7 and 2% by weight, based on the dry mordenite weight.

3. Catalyst according to one of the claims 1 and 2, characterized in that
– said mordenite is such that its skeleton Si/Al atomic ratio is between 6 and 9.5 and
– its sodium weight content is below 1000 ppm.

4. Catalyst according to one of the claims 1 to 3 in which the said metal is chosen from the group formed by platinum and palladium.

5. Catalyst according to one of the claims 1 to 4, characterized in that it also contains a matrix.

6. Process for the preparation of a mordenite contained in a catalyst according to one of the claims 1 to 5 from a so-called small pore mordenite, whose skeleton Si/Al atomic ratio is between 4.5 and 6.5, whose sodium weight content is between 4 and 6.5%, whose unit cell volume is between 2.77 and 2.80 nm$^3$, said small pore mordenite only adsorbing molecules with a kinetic diameter below approximately $4.4 \times 10^{-10}$m, the process being characterized In that:

a) there is at least one exchange of the sodium cations of the small pore mordenite by ammonium cations,

b) the solid obtained in stage a) is subject to a calcination under a dry air stream, whose water weight content is below 1%, under an air flow rate between 0.5 and 10 litres/hour/gramme of solid, at a temperature between approximately 450 and 650°C, with a temperature rise rate between 2 and 8°C/minute, the final calcination temperature being maintained for approximately 0.5 to approximately 5 hours.

7. Process according to claim 6 in which the solid obtained in stage b) is heated in a mineral or organic acid solution with a normallty below 2 N, at a temperature below approximately 100°C, with a $H_s^+/Al_z$ ratio between 8.5 and 11.5, in which $H_s^+$ is the number of proton moles i solution and $Al_z$ the number of aluminium cation moles in the mordenite.

8. Process for the preparation of a mordenite contained in a catalyst according to one of the claim 1 to 5 from a small pore mordenite, whose skeleton Si/Al atomic ratio is between 4.5 and 6.5, whose sodium weight content is between 4 and 6.5%, whose unit cell volume is between 2.77 and 2.80 nm$^3$, said small pore mordenite only adsorbing molecules with a kinetic diameter below approximately $4.4 \times 10^{-10}$ m, the process being characterized in that:

a) there is at least one exchange of the sodium cations of the small pore mordenite by ammonium cations,

b) the solid obtained in stage a) is heated to a temperature above approximately 70°C in a solution of a mineral or organic acid with a normality between 0.1 and 5N, with a $H_s^+/Al_z$ ratio between 5 and 20, in which $H_s^+$ is the number of proton moles in the solution and $Al_z$ the number of aluminium cation moles in the mordenite.

9. Use of a catalyst according to one of the claims 1 to 5 in isomerization reactions of a $C_8$ aromatic fraction.

## Patentansprüche

1. Katalysator, enthaltend einen Mordenit und mindestens ein Metall der Gruppe VIII des Periodischen Systems der Elemente, dadurch gekennzeichnet, daß es sich bei dem Mordenit um einen solchen handelt,
– dessen Atomverhältnis Si/Al im Gerüst zwischen 6 und 10,5 liegt,
– dessen Gewichtsgehalt an Natrium unterhalb 2 000 ppm liegt,
– dessen Elementargittervolumen zwischen 2,73 und 2,78 nm$^3$ liegt,
– dessen Adsorptionsvermögen für Benzol zwischen 4 und 10 Gew.-%, bezogen auf das Gewicht des trockenen Mordenits, liegt, und
– dessen Adsorptionsvermögen für 1,3,5-Trimethylbenzol zwischen 0,5 und 2,5 Gew.-%, bezogen auf das Gewicht des trockenen Mordenits, liegt.

2. Katalysator nach Anspruch 1, dadurch gekennzeichnet, daß es sich bei dem Mordenit um einen solchen handelt,
– dessen Adsorptionsvermögen für Benzol zwischen 5 und 9 Gew.-%, bezogen auf das Gewicht des trockenen Mordenits, liegt, und
– dessen Adsorptionsvermögen für 1,3,5-Trimethylbenzol zwischen 0,7 und 2 Gew.-%, bezogen auf das Gewicht des trockenen Mordenits, liegt.

3. Katalysator nach einem der Ansprüche 1 und 2, dadurch gekennzeichnet, daß es sich bei dem Mordenit um einen solchen handelt,
– dessen Atomverhältnis Si/Al im Gerüst zwischen 6 und 9,5 liegt und
– dessen Gewichtsgehalt an Natrium unterhalb 1 000 ppm liegt.

4. Katalysator nach einem der Ansprüche 1 bis 3, in dem das Metall ausgewählt wird aus der Gruppe Platin und Palladium.

5. Katalysator nach einem der Ansprüche 1 bis 4, dadurch gekennzeichnet, daß er außerdem eine Matrix enthält.

6. Verfahren zur Herstellung eines Mordenits, der in einem Katalysator nach einem der Ansprüche 1 bis 5 enthalten ist, aus einem "kleinporigen" Mordenit, dessen Atomverhältnis Si/Al im Gerüst zwischen 4,5 und 6,5 liegt, dessen Gewichtsgehalt an Natrium zwischen 4 und 6,5 % liegt, dessen Elementargittervolumen zwischen 2,77 und 2,80 nm$^3$ liegt, wobei der "kleinporige" Mordenit nur Moleküle mit einem kinetischen Durchmesser von weniger als etwa $4,4 \times 10^{-10}$ m adsorbiert, das dadurch gekennzeichnet ist, daß man

(a) mindestens einmal einen Austausch der Natrium-Kationen des "kleinporigen" Mordenits gegen Ammoniumkationen durchführt und

(b) den in der Stufe (a) erhaltenen Feststoff einer Calcinierung unter einem trockenen Luftstrom, dessen Gewichtsgehalt an Wasser unter 1 % liegt, bei einer Luftzuführungsmenge zwischen 0,5 und 10 l pro Stunde und pro g Feststoff bei einer Temperatur zwischen etwa 450 und etwa 650°C mit einer Temperatursteigerungsgeschwindigkeit zwischen 2 und 8°C pro Minute unterwirft, wobei die Calcinierungs-Endtemperatur etwa 0,5 bis etwa 5 Stunden lang aufrechterhalten wird.

7. Verfahren nach Anspruch 6, in dem der in der Stufe (b) erhaltene Feststoff in einer Lösung einer Mineralsäure oder einer organischen Säure mit einer Normalität von weniger als 2 N auf eine Temperatur von etwa 100°C bei einem Verhältnis $H_s^+/Al_z$ zwischen 8,5 und 11,5 erhitzt wird, wobei $H_s^+$ für die Anzahl der Mole an Protonen in der Lösung und $Al_z$ für die Anzahl der Mole an Aluminiumkationen in dem Mordenit stehen.

8. Verfahren zur Herstellung eines Mordenits, wie er in einem Katalysator nach einem der Ansprüche 1 bis 5 enthalten ist, aus einem "kleinporigen" Mordenit, dessen Atomverhältnis Si/Al im Gerüst zwischen 4,5 und 6,5 liegt, dessen Gewichtsgehalt an Natrium zwischen 4 und 6,5 % liegt, dessen Elementargittervolumen zwischen 2,77 und 2,80 nm³ liegt, wobei der "kleinporige" Mordenit nur Moleküle mit einem kinetischen Durchmesser von weniger als etwa 4,4 x $10^{-10}$ m adsorbiert, das dadurch gekennzeichnet ist, daß

(a) man mindestens einen Austausch der Natriumkationen des "kleinporigen" Mordenits gegen Ammoniumkationen durchführt und

(b) man den in der Stufe (a) erhaltenen Feststoff auf eine Temperatur von oberhalb etwa 70°C in einer Lösung einer Mineralsäure oder einer organischen Säure mit einer Normalität zwischen 0,1 und 5 N bei einem Verhältnis $H_s^+/Al_z$ zwischen 5 und 20 erwärmt, wobei $H_s^+$ für die Anzahl der Mole an Protonen in der Lösung und $Al_z$ für die Anzahl der Mole an Aluminiumkationen in dem Mordenit stehen.

9. Verwendung eines Katalysators nach einem der Ansprüche 1 bis 5 in den Reaktionen zur Isomerisierung einer aromatischen $C_8$-Fraktion.